# EUROPÄISCHE PATENTSCHRIFT

(11) **EP 1 706 074 B1**
(45) Veröffentlichungstag und Bekanntmachung des Hinweises auf die Patenterteilung: **26.12.2012**
(21) Anmeldenummer: 05700306.3
(22) Anmeldetag: 13.01.2005
(51) Int. Cl.: A61F 2/40

(54) **SCHAFTFREIE SCHULTERGELENKSPROTHESE**
SHOULDER JOINT PROSTHESIS WITHOUT SHAFT
PROTHÈSE D'ARTICULATION DE L'ÉPAULE SANS TIGE

(30) Priorität: 22.01.2004 CH 822004
(43) Veröffentlichungstag der Anmeldung: 04.10.2006
(73) Patentinhaber: SQ Products AG, 6312 Steinhausen (CH)
(72) Erfinder: HABERMEYER, Peter, 69120 Heidelberg (DE); KÄLIN, Peter, 6314 Unterägeri (CH)
(74) Vertreter: Troesch, Jacques J.
(86) Internationale Anmeldenummer: PCT/CH2005/000011
(87) Internationale Veröffentlichungsnummer: WO 2005/070345

(56) Entgegenhaltungen:
- EP-A- 0 664 108
- EP-A- 1 125 565
- DE-C1- 10 123 517
- GB-A- 2 007 980

## Beschreibung

Die vorliegende Erfindung betrifft eine schaftfreie Schultergelenkprothese gemäss dem Oberbegriff nach Anspruch 1.

Aus dem Stand der Technik sind eine Reihe von Schultergelenkprothesen bekannt, wie beispielsweise aus der EP 0 664 108, der EP 1 125 565 und der DE 101 23 517. In allen Dokumenten werden Prothesen beschrieben, welche einen Schaft aufweisen für die Verankerung der Prothese im Oberarmknochen.

Insbesondere bei jungen Patienten oder Patienten, bei welchen die Knochensubstanz gut erhalten ist, wird darauf geachtet, dass beim Anordnen einer Schulterprothese möglichst wenig Knochensubstanz verloren geht. Wichtig ist auch, dass die Beweglichkeit möglichst erhalten bleibt bei gleichzeitiger Gelenkstabilität.

Bekannt hierzu sind sogenannte Cup-Schulterprothesen, welche u.a. von den Firmen Centerpulse und Biomet Orthopedics angeboten werden. Bei diesen sogenannten Cup-Schulterprothesen wird die Kopfkomponente mittels eines mittig innerhalb des kappenartigen Kopfes angeordneten Schaftes am Gelenkkopfknochen mit oder ohne Knochenzement fixiert. Durch den somit weitgehend erhaltenen Gelenkkopf ist die Zugänglichkeit zur Gelenkpfanne stark erschwert. Damit unterbleibt in der Regel eine notwendige Pfannenversorgung.

Es ist eine Aufgabe der vorliegenden Erfindung, eine Humeruskopf- bzw. Schultergelenkprothese vorzuschlagen, welche insbesondere geeignet ist bei Patienten mit gut erhaltener Knochensubstanz, sowohl den Einbau einer Gelenkpfanne als auch den zementfreien Gelenkkopfersatz zu ermöglichen. Erfindungsgemäss wird die gestellte Aufgabe mittels einer schaftfreien Schultergelenkprothese gemäss dem Wortlaut nach Anspruch 1 gelöst.

Im Gegensatz zu den bekannten sogenannten Cup-Schulterprothesen wird erfindungsgemäss vorgeschlagen, den Gelenkkopf nicht mittels eines fest am Kopf angeordneten Schafts am Oberarm- oder Schulterknochen zu befestigen, sondern zunächst ein Befestigungsorgan anzuordnen, auf welches der Humeruskopf bzw. der Gelenkkopf aufgesetzt wird. Eine vorbekannte Cup-Prothese ist in der GB-A-2 007 980 erwähnt und im Vorzeichen von Patentanspruch 1 gewürdigt

Vorzugsweise ist das Befestigungsorgan mindestens zweiteilig ausgebildet, bestehend aus einer sogenannten Druckscheibe, ggf. auch zu bezeichnen als Positionsscheibe, und einer Hohlschraube, mittels welcher die Scheibe auf dem Knochen fixiert wird. Somit wird im Gegensatz zu den Cup-Schulterprothesen im vorliegenden Fall nicht nur der Knorpel bis zum subchondralen Knochen abgetragen, sondern der ganze Kopf entlang des Collum anatonicum reseziert. Der Kopfersatz, vorzugsweise bestehend aus der erwähnten Hohlschraube, der Druckscheibe und dem Gelenkkopf wird mit der Hohlschraube zementfrei im Humerus fixiert. Die erfindungsgemäss vorgeschlagene Prothese ist sowohl für den hemiprothetischen, als auch für den totalen Gelenkersatz geeignet.

Die erfindungsgemäss schaftfreie Humeruskopfprothese liegt mit der erwähnten Druckscheibe auf der Resektionsfläche auf. Die Scheibe wird jeweils so gross gewählt, dass rundherum eine kortikale Abstützung entsteht. Die Druckscheibe wird mit der erwähnten Hohlschraube in der Spongiosa befestigt. Dadurch erfolgt unter Last eine Krafteinleitung sowohl auf die Kortikalis, als auch auf die Spongiosa.

Klassische Primär-Schaftprothesen erfordern eine Resektion des Humeruskopfes entlang des anatomischen Halses. Bei der schaftfreien erfindungsgemäss vorgeschlagenen Humeruskopfprothese wird ebenfalls an dieser Stelle reseziert. Bei einem allenfalls notwendigen Revisionseingriff ermöglicht dies ohne Resektionskorrekturen den späteren Einsatz einer klassischen Primär-Schaftprothese.

Im Vergleich zu den oben erwähnten aus dem Stand der Technik bekannten Cup-Prothesen zeigt sich, dass die Gelenkkopfkappe bis unterhalb des anatomischen Halses ragt. Im Falle eines Cup-Ausbaus muss dann die Resektion unterhalb des anatomischen Halses erfolgen, was zu tief ist für den Einbau von klassischen Stielprothesen.

Ein weiterer Vorteil der schaftfreien erfindungsgemäss vorgeschlagenen Humeruskopfprothese liegt darin, dass eine Implantation unabhängig von der Humerusschaftgeometrie möglich ist. Speziell im Falle von veralteten Oberarmfrakturen kommt es oft zu einer Verschiebung der gebrochenen Kopfkalotte gegenüber dem Humerus. Dabei ist der Humerus oft nach anterior und nach medial verschoben und das Kopfsegment nach posterior und nach lateral versetzt. Der Einsatz von konventionellen Schaftprothesen kann dadurch stark erschwert werden, da durch den Versatz das Einführen des Schaftes in den Markraum beeinträchtigt wird und der mediale Offset zu gross sein kann.

Schliesslich als Vorteile der erfindungsgemäss vorgeschlagenen schaftfreien Humeruskopfprothese seien erwähnt die relativ einfache Handhabung und die sichere und bewährte zementfreie Verankerung mit der erfindungsgemäss vorgeschlagenen Hohlschraube. Zudem ist es möglich, ein umfangreiches Sortiment an Implantatgrössen bereitzuhalten und somit anatomisch korrekte Wiederherstellung des Gelenks durch modulare Bauweise zu ermöglichen.

Die Erfindung wird nun beispielsweise und unter Bezug auf die beigefügten Figuren näher erläutert.

Dabei zeigen:
- Fig. 1: in Perspektive im zusammengesetzten Zustand eine erfindungsgemäss schaftfreie Humeruskopfprothese;
- Fig. 2: die Prothese aus Fig. 1 in Perspektive und auseinander gezogenem Zustand;
- Fig. 3: den Gelenkkopf aus Fig. 2 in Perspektive, jedoch derart gedreht, dass von der Gelenkkopf entgegengesetzten Seite sichtbar;
- Fig. 4: einen Längsschnitt durch eine erfindungsgemässe Humeruskopfprothese und
- Fig. 5: in auseinander gezogenem, zerlegtem Zustand im Längsschnitt die Prothese aus Fig. 4.

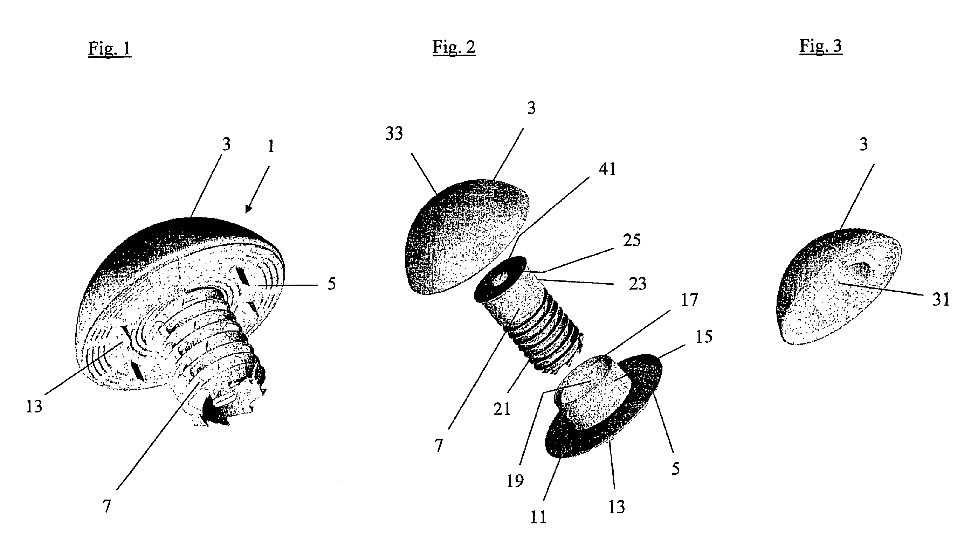

Fig. 1 zeigt in Perspektive seitlich von unten gesehen eine erfindungsgemässe Humeruskopfprothese 1, aufweisend einen Gelenkkopf 3, welcher auf einer Druckscheibe 5 aufgesetzt und an dieser annähernd anliegend angeordnet ist. Mittig durch die Druckscheibe 5 hindurchragend ist eine Hohlschraube 7 angeordnet, vorgesehen um den Gelenkkopf 3 mit Druckscheibe 5 im bzw. am Knochen festzulegen. Ebenfalls erkennbar sind von der Druckscheibe 5 nach unten vorstehende Fixierhaken oder -nasen 13, um die Druckscheibe drehfest am Knochen festzulegen.

Fig. 2 zeigt die Prothese aus Fig. 1 erneut in Perspektive, jedoch auseinander gezogen und in Einzelteilen dargestellt. Dabei erkennbar ist der Gelenkkopf 3 mit der wenigstens nahezu kugelförmig ausgebildeten Gelenkoberfläche 33. Die Hohlschraube 7 weist ein Gewinde 21 auf, um die Hohlschraube im Knochen zu fixieren. Dabei wird die Hohlschraube 7 durch ein mittiges Loch 19 der Druckscheibe 5 hindurch in den Knochen getrieben. Weiter weist die Hohlschraube 7 einen wenigstens nahezu glattwandigen Schaft 23 auf, welcher Innen anliegend an einen Kragen 15 vorstehend von der Druckscheibe 5 beim Eindrehen der Hohlschraube 7 in den Knochen zu liegen kommt. Endständig an der Hohlschraube 7 angeordnet ist ein Halterand 25, welcher abgeschrägt ausgebildet ist, um an einem ebenfalls abgeschrägt oder konisch ausgebildeten Auflagerand 17 endständig am Kragen 15 ausgebildet aufzuliegen.

Schliesslich dargestellt ist die Druckscheibe 5 mit einer ringförmig ausgebildeten Partie 11, an deren Unterseite die erwähnten Fixierhaken 13 angeordnet sind.

Fig. 3 zeigt den Gelenkkopf 3 in Perspektive, jedoch gegenüber der Darstellung in Fig. 2 gedreht, sodass er von der Unterseite sichtbar ist. Deutlich erkennbar ist eine hohlkonusförmig ausgebildete Aufnahme 31, welche geeignet ist zur Aufnahme des kragenartigen Vorsprunges 15 der Druckscheibe 5.

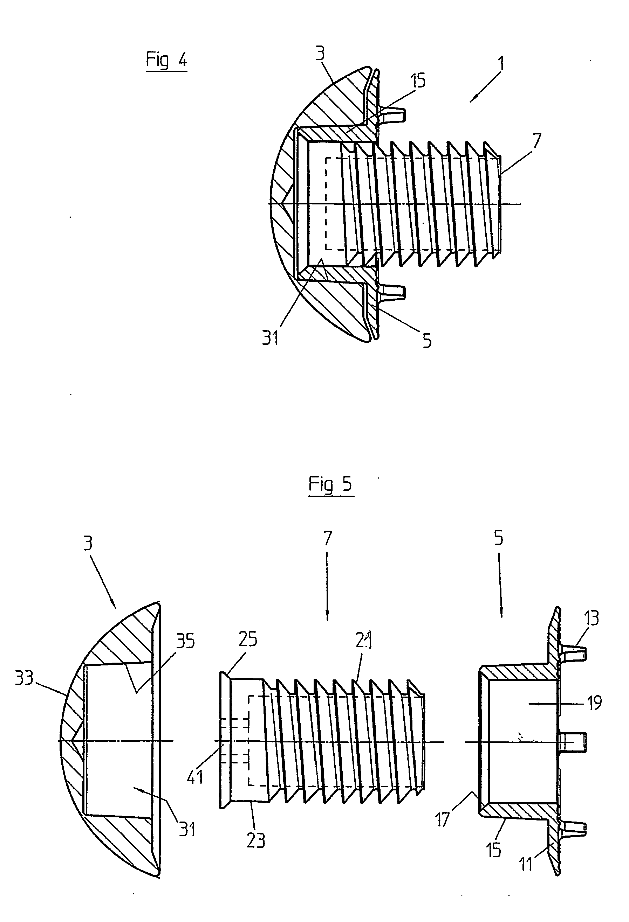

In Fig. 4 ist die erfindungsgemäss definierte Humeruskopfprothese im Längsschnitt dargestellt, wobei nun deutlich erkennbar ist, wie der Rand oder Kragen 15 der Positions- oder Druckscheibe 5 in der hohlkonusförmigen Aufnahme 31 des Gelenkkopfes 3 angeordnet ist.

Fig. 5 schliesslich zeigt erneut die erfindungsgemässe Gelenkprothese zerlegt bzw. in auseinander gezogenem Zustand, darstellend die drei Einzelteile. Aus Fig. 5 ist zunächst deutlich erkennbar, dass der endständige Auflagerand des Kragens 15 konisch bzw. abgeschrägt ausgebildet ist, wie entsprechend der seitlich von der Hohlschraube 7 vorstehende, endständig ausgebildete Halterand 25. Diese konische bzw. abgeschrägte Ausbildung der beiden Bereiche 17 und 25 garantiert ein positionsgetreues Einsetzen der Hohlschraube 7 in das mittige Loch 19 der Druckscheibe 5. Weiter erkennbar ist, dass auch der Rand 15 der Scheibe 5, wie auch die innere Wandung 35 der rund ausgebildeten Aufnahme 31, je leicht abgeschrägt bzw. konisch ausgebildet sind, um ein festes Aufsetzen des Gelenkkopfes 3 über dem Kragen 15 der Scheibe 5 zu ermöglichen. In der Praxis hat es sich gezeigt, dass der Gelenkkopf 3 beim Aufsetzen lediglich mittels eines leichten Schlages aus dem Gelenkkopf derart festgelegt werden kann, dass ein späteres Lösen bei Bewegen des Oberarmes unmöglich wird.

Das Anordnen der erfindungsgemässen Prothese ist an sich sehr einfach, indem zunächst auf den vorab vorbereiteten Knochen die Positions- oder Druckscheibe 5 angelegt bzw. positioniert wird. Durch die nach unten vorstehenden Vorsprünge bzw. Haken 13 ist eine provisorische positionstreue Fixierung der Scheibe am Knochen möglich. Nun wird die Hohlschraube 7 durch das mittige Loch 19 eingedreht, wobei dies beispielsweise mittels eines sogenannten Imbusschlüssels erfolgen kann, welcher mittig an einem inständigen Loch 41 der Hohlschraube 7 eingreift.

Nach erfolgtem Eindrehen der Hohlschraube 7, derartig, dass der Halterand 25 am konischen Auflagerand 17 anliegt, kann nun der Gelenkkopf 3 angeordnet werden, indem dieser über dem Kragen 15 angelegt wird. Zur definitiven Fixierung ist schliesslich beispielsweise ein leichter Schlag auf die äussere Gelenkkontur 33 nötig.

Bei der unter Bezug auf die Figuren 1 bis 5 dargestellten erfindungsgemässen Prothese handelt es sich selbstverständlich lediglich um ein Beispiel, welches auf x-beliebige Art und Weise abgeändert, modifiziert oder durch weitere Elemente ergänzt werden kann. Insbesondere bezieht sich die Darstellung lediglich auf die Gelenkkopfprothese und auf die Darstellung der entsprechenden Gelenkpfanne wurde verzichtet, da Gelenkpfannen im Prothesenbau bestens bekannt sind. So ist es beispielsweise möglich, eine Gelenkpfanne ähnlich, beispielsweise an einem Schulterknochen anzuordnen, indem eine Gelenkpfanne mittels Konusteil und Hohlschraube in der Scapula bzw. dem Schulterblattknochen befestigt werden kann. Ebenfalls die Materialwahl für die Herstellung eines erfindungsgemässen Gelenkkopfes ist an sich kein Thema der vorliegenden Erfindung. Verwendet werden beispielsweise Legierungen auf Kobaltchrom-Basis. Für die übrigen Teile der erfindungsgemässen Prothese eignen sich beispielsweise Metalllegierungen auf Titan-Basis. Auch die Geometrie der erfindungsgemässen Prothese ist an sich kein Thema der vorliegenden Erfindung, indem diese einerseits an die spezifischen Verhältnisse des jeweiligen Schultergelenkes anzupassen ist und im Übrigen vorzugsweise weitgehendst kugelförmig ausgebildet ist. Gemäss der vorliegenden Erfindung ist es dabei vorteilhaft, dass der Gelenkkopf bzw. dessen Oberfläche kleiner als halbkugelförmig ausgebildet sein kann, wodurch sowohl Handhabung, als auch ein späterer Ersatz vereinfacht werden.

Auch die Materialien der Befestigungsorgane sind an sich bekannt, wie beispielsweise vorzugsweise Legierungen auf Basis von Titan. Grundsätzlich gilt, dass Dimensionen der Einzelteile, wie insbesondere des Humeruskopfes selbst und der dazugehörigen Gelenkpfanne, abgeändert bzw. variiert werden können und auch die für die Prothese verwendeten Materialien können entsprechend den Bedürfnissen und auch Neuentwicklungen in den Materialwissenschaften entsprechend angepasst werden.

## Patentansprüche

1. Schaftfreie Schultergelenkprothese, bestehend aus einer mindestens zweiteiligen Humeruskopfprothese, bestehend aus einem Gelenkkopf (3) und einem Befestigungsorgan (5, 7), **dadurch gekennzeichnet**, daβß das Befestigungsorgan aus einem Positionsorgan (5) und einem Verankerungsorgan (7), besteht wobei das Positionsorgan eine Festhaltepartie (15) zum aufsetzenden Festlegen des Gelenkkopfs aufweist, und das Verankerungsorgan (7) in der Form einer Hohlschraube zum zementfreien Verankern des Positionsorgans in der Spongiosa vorliegt.

2. Prothese nach Anspruch 1, **dadurch gekennzeichnet, dass** das Befestigungsorgan mindestens zweiteilig ausgebildet ist, aufweisend ein scheibenartiges, ein mittiges Loch aufweisendes Positionsorgan (5), sowie ein Verankerungsorgan (7), vorgesehen, um durch das mittige Loch (19) hindurch das Positionsorgan (5) am Knochen fest anzuordnen.

3. Prothese nach einem der Ansprüche 1 oder 2, **dadurch gekennzeichnet, dass** der Gelenkkopf (3) eine wenigstens nahezu kugelförmige Oberfläche (33) aufweist, entsprechend einem Kugelabschnitt mit einem Öffnungswinkel von < 180°.

4. Prothese nach einem der Ansprüche 2 oder 3, **dadurch gekennzeichnet, dass** das scheibenartige Positionsorgan einen wenigstens nahezu kreisrunden vorstehenden Kragen (15) aufweist mit einem endständig angeordneten, vorzugsweise konisch ausgebildeten Auflagerand, und dass am Verankerungsorgan bzw. an der Hohlschraube (7) endständig nach Aussen hin vorstehend entsprechend dem Auflagerand ein abgeschrägter Halterand (25) ausgebildet ist, um auf dem Auflagerand (17) im Innern des Kragens (15) auf- bzw. anzuliegen.

5. Prothese nach einem der Ansprüche 1 bis 4, **dadurch gekennzeichnet, dass** der Gelenkkopf (3) auf der, der Kugeloberfläche (33) entgegengesetzten Seite eine wenigstens nahezu hohlzylinderförmig ausgebildete Aufnahme (31) aufweist, vorgesehen, um den Gelenkkopf auf dem Befestigungsorgan aufgesetzt anzuordnen.

6. Prothese nach Anspruch 5, **dadurch gekennzeichnet, dass** die Wandung (35) der hohlzylinderförmigen Aufnahme (31) und die äussere Oberfläche des Kragens (15) der Positions- oder Druckscheibe (5) je leicht konisch verlaufend bzw. abgeschrägt ausgebildet sind, derart, um ein formschlüssiges und positionstreues Aufsetzen des Gelenkkopfes über den Kragen (15) zu ermöglichen.

## Claims

1. Shaftless shoulder joint prosthesis consisting of an at least two-part humeral head prosthesis consisting of a joint head (3) and an attachment organ (5, 7), **characterised in that** the attachment organ consists of a positioning organ (5) and an anchoring organ (7), wherein the positioning organ comprises an attachment part (15) for additionally fixing the joint head, and the anchoring organ (7) is in the form of a hollow screw for cement-free anchoring of the positioning organ in the cancellous bone.

2. Prosthesis according to claim 1, **characterised in that** the fixing organ is formed of at least two pieces, comprising a disk-like positioning organ (5) comprising a central hole, as well as an anchoring organ (7) provided so as to fixedly arrange the positioning organ (5) on the bone through the central hole (19).

3. Prosthesis according to one of claims 1 or 2, **characterised in that** the joint head (3) features a substantially spherical surface (33), conforming to a spherical section with an opening angle of < 180°.

4. Prosthesis according to one of claims 2 or 3, **characterised in that** the disk-like positioning organ features an at least substantially circular protruding collar (15) with a terminally-arranged preferably conically-formed supporting edge, and **in that** a beveled retaining flange (25) conforming to the supporting edge is formed terminally on the anchoring organ and/or the hollow screw (7), protruding outwardly so as to abut the supporting edge (17) on the inside of the collar (15).

5. Prosthesis according to one of claims 1 to 4, **characterised in that** the joint head (3) features an at least substantially cylindrically-shaped recess (31) on its side opposite to the spherical surface (33), provided so as to mount the joint head on the fixing organ.

6. Prosthesis according to claim 5, **characterised in that** the wall (35) of the hollow-cylindrically formed recess (31) and the outer surface of the collar (15) of the positioning or pressure disk (5) are each formed lightly conical and/or tapered so as to permit a form-fitted and positionally-accurate placement of the joint head over the collar (15).

## Revendications

1. Prothèse d'épaule sans arbre se composant d'une prothèse de tête d'humérus ayant au moins deux parties, se composant d'une tête d'articulation (3) et d'un organe de fixation (5, 7), **caractérisée en ce que** l'organe de fixation se compose d'un organe de positionnement (5) et d'un organe d'ancrage (7), l'organe de positionnement comprenant une partie d'attache (15) pour attacher la tête d'articulation, et l'organe d'ancrage (7) étant en forme d'une vis creuse pour l'ancrage sans cément de l'organe de positionnement dans l'os spongieux.

2. Prothèse selon la revendication 1, **caractérisée en ce que** l'organe de fixation est formé d'au moins deux pièces, comprend un organe de position (5) en forme de disque et ayant une ouverture centrale, et comprend ainsi un organe d'ancrage (7) prévu pour fixer l'organe de positionnement à travers de l'ouverture centrale (19).

3. Prothèse selon l'une des revendications 1 ou 2, **caractérisée en ce que** la tête d'articulation (3) présente une surface (33) au moins substantiellement sphérique, conformant à une section de sphère ayant un angle d'ouverture de moins de 180°.

4. Prothèse selon l'une des revendications 2 ou 3, **caractérisée en ce que** l'organe de positionnement en forme de disque comprend un collier au moins substantiellement circulaire ayant un rebord de support préférablement conique agencé terminalement, et **en ce qu'**un rebord de retenue (25) conformant au rebord de support est formé terminalement sur l'organe d'ancrage et/ou sur la vis creuse, s'étendant en saillie vers l'extérieur, afin de contacter le rebord de support (17) à l'intérieur du collier (15).

5. Prothèse selon l'une des revendications 1 à 4, **caractérisée en ce que** la tête d'articulation (3) présente une cavité au moins substantiellement cylindrique située sur le côté opposé à la surface sphérique, prévue pour supporter la tête d'articulation sur l'organe de fixation.

6. Prothèse selon la revendication 5, **caractérisée en ce que** la paroi (35) de la cavité cylindrique creuse (31) et la surface extérieure du collier (15) du disque de positionnement ou de pression (5) sont chacune formée légèrement conique et/ou effilée, de telle manière à permettre un positionnement emmanché et précis de la tête d'articulation sur le collier (15).
